# EUROPEAN PATENT SPECIFICATION

(11) **EP 3 103 438 B1**
(45) Date of publication and mention of the grant of the patent: **14.11.2018**
(21) Application number: 15171692.5
(22) Date of filing: 11.06.2015
(51) Int. Cl.: A61K 9/00, A61K 47/20, A61K 9/08, A61K 36/00

(54) **TOPICAL NASAL COMPOSITIONS CONTAINING XYLOGLUCANS FOR USE AS DECONGESTANS**
TOPISCHE NASALE ZUSAMMENSETZUNGEN MIT XYLOGLUCANEN ZUR VERWENDUNG ALS ABSCHWELLENDES MITTEL
COMPOSITIONS NASALES TOPIQUES CONTENANT DES XYLOGLUCANES POUR UTILISATION COMME DÉCONGESTIONNANTS

(43) Date of publication of application: 14.12.2016
(73) Proprietor: Novintethical Pharma SA, 6900 Lugano (CH)
(72) Inventor: DI FULVIO, Marco, 6915 LUGANO (CH); ALONSO COHEN, Miguel Angel, 08034 BARCELLONA (ES)
(74) Representative: Bianchetti Bracco Minoja S.r.l.

(56) References cited:
- WO-A1-2013/132487
- CN-A- 104 069 156
- NAKAMURA FUMIHIKO ET AL: "Evaluation of halopredone acetate ground mixture using experimental allergic rhinitis models in rats", YAKUZAIGAKU - JOURNAL OF PHARMACEUTICAL SCIENCE AND TECHNOLOGY, YAKUJI NIPPO-SHA, TOKYO, JP, vol. 59, no. 4, 1 December 1999 (1999-12-01), pages 163-168, XP009176208, ISSN: 0372-7629
- HENSEL A: "Xyloglucan--structure, genesis, and functions of a widely distributed substance group", PHARMAZIE IN UNSERER ZEIT, VCH VERLAGSGESELLSCHAFT, WEINHEIM, DE, vol. 22, no. 4, 1 September 1993 (1993-09-01), pages 228-234, XP002486565, ISSN: 0048-3664, DOI: 10.1002/PAUZ.19930220426

## Description

The invention relates to xyloglucans or extracts containing them in combination with pea protein and optionally with one or more active ingredients and/or excipients, wherein xyloglucans and pea protein are in 1:1 weight ratio, for use in the topical intranasal treatment of nasal congestion.

### Prior art

Nasal congestion is the result of inflammation and swelling of the tissues lining the nasal passages, and can have multiple causes, such as infections, allergies, or structural causes such as deviation of the nasal septum. In particular, nasal congestion is associated with rhinitis and sinusitis.

Rhinitis is an inflammation of the nasal mucosa caused, for example, by colds, influenza or allergies. Rhinitis can be characterised by one or more symptoms such as rhinorrhoea, sneezing, nasal congestion and increased nasal secretions. Rhinitis can include acute rhinitis, rhinitis, allergic rhinitis, seasonal allergic rhinitis, chronic allergic rhinitis, vasomotor rhinitis, infectious rhinitis and atrophic rhinitis. Sinusitis is an inflammation of the paranasal sinuses, which may result from infection (e.g. bacterial, fungal or viral infection), or have allergic or autoimmune causes.

The medicaments most commonly used to treat rhinitis and nasal congestion are anti-H1 antihistamines, which inhibit the action of histamine by blocking the histamine bond with the H1 receptors or the atypical antihistamines that inhibit the enzymatic activity of histidine decarboxylase, which catalyses the transformation of histidine to histamine. Antihistamines are effective against symptoms mediated by histamine, such as rhinorrhoea, sneezing, nasal itching and eye symptoms, but less effective against nasal congestion.

The side effects of the first-generation oral anti-H1 antihistamines include sedation and adverse effects on the central nervous system (alertness disorders, drowsiness, and effects on driving vehicles). These disorders may be exacerbated by alcohol and other sedatives. Moreover, anti-H1 antihistamines can interact with some medicaments, plant products, nutritional and diet supplements.

Intranasal corticosteroids remain the preferred treatment for nasal congestion, but side effects may occur in about 5% of patients, such as formation of scabs, dryness and minor epistaxis, which may lead to discontinuance of the treatment. Children treated regularly for a year with nasal beclomethasone exhibited a slight growth delay.

Another group of medicaments used to treat nasal congestion comprises decongestants such as pseudoephedrine and phenylephrine. Oral and intranasal decongestants can be used to treat nasal congestion associated with allergic rhinitis. However, the use of decongestants administered per os or as a nasal spray is limited by side effects such as a rebound effect on congestion, the possibility of inducing irreversible tissue hypertrophy, and a reduction in the duration of the effect after 10 days' use. For example, rhinitis medicamentosa is induced or aggravated by excessive use of topical decongestants with or without preservatives.

Antileukotrienes are a novel class of drugs for the treatment of allergic rhinitis and asthma. Their efficacy is comparable to that of oral antihistamines, although they appear to have a greater effect on nasal obstruction. Other products used to treat rhinitis, such as antibiotics, anticholinergics and chromones, do not have a decongestant action. Instillation of saline solution, in particular in the paediatric field (Chirico G et al., Minerva Pediatrica, 62(5), 2010- 499-505), is also common, but although it temporarily alleviates the symptoms, it is obviously merely a short-term solution of limited efficacy.

WO 2013/132487 discloses nose drops of extract containing xyloglucans (specifically pomegranate extract) for use in treating rhinitis or sinusitis, optionally with further actives.

There is consequently still a need for further treatments to replace or accompany those available today.

### Disclosure of the invention

The invention therefore relates to xyloglucans or extracts containing them in combination with pea protein and optionally with one or more active ingredients and/or excipients, wherein xyloglucans and pea protein are in 1:1 weight ratio, for use in the topical intranasal treatment of nasal congestion.

Xyloglucans are molecules consisting of a linear backbone of β-1,4-glucans with short side branches. They bond due to the xylose bonded to oxygen in the 6 position of the sugar. Said side chains can also contain other sugars such as arabinose and fucose.

Xyloglucans belong to the hemicellulose family, which combines with cellulose in the cell wall of the higher plants. A particularly rich source of xyloglucan is the seeds of tamarind (*Tamarindus indica*), a tropical tree originating from East Africa.

Xyloglucan-rich tamarind seed extracts are known and have been used in the medical field mainly as viscosity-controlling agents in ophthalmic compositions (US 6 056 950), mucoadhesive agents (WO2006131262), artificial tears (WO2009/044423), anti-infective agents (WO2011147767) and anti-inflammatory agents (WO2011147768).

Nakamura F et al., Yakuzaigaku, 59(4) 1999, 163-168 described formulations of steroidal anti-inflammatory drugs containing tamarind gum as excipient.

Xyloglucans extracted from Tamarindus indica seeds, available on the market, for example, from Indena (Italy) (Xilogel®) and DSP Gokyo Food & Chemical (Japan) (Glyloid®), are preferred. The weight-average molecular weight Mw of the preferred xyloglucan is about 650,000 Da, determined by light diffusion with He-Ne laser source λ=632.8nm.

The topical nasal compositions according to the invention can also include one or more constituents, active ingredients and/or excipients. For example, the compositions according to the invention can include antibiotics, antivirals, antiinflammatories and decongestants as further active ingredients.

The compositions according to the invention can also contain excipients and/or functional agents (moisturising, mucoadhesive or film-forming agents, preservatives, etc.).

Examples of said further ingredients include substances such as sucralfate, natural and synthetic polysaccharides such as pectins, chitosan (animal or vegetable), hyaluronic acid, methylsulphonylmethane (MSM), guar gum, agar agar, xanthan gum, animal proteins such as gelatin, albumin, ovalbumin, casein, plant proteins such as pea protein, soya protein, cellulose and hemicellulose and derivatives such as hydroxypropylcellulose, carrageenans, carbomers, and crosslinking/polymerising compounds such as ferulic acid; polyphenols, such as gall polyphenols, grape-seed polyphenols, mono- and oligosaccharides.

Methylsulphonylmethane is particularly preferred. Combined use with xyloglucans obtained from plant proteins, in particular pea protein and/or soya protein, is even more preferred.

The claims demand a combination of xyloglucans and pea protein in a 1:1 weight ratio.

The weight ratio of xyloglucan to protein mainly ranges between 1:0.5 and 1:30, the ratio 1:1 being preferred, and in the case of pea protein being obligatory according to the claims. Said associations increase some of the characteristics of xyloglucan, such as the ability to retain water molecules and swell for a longer time, with a more efficient moisturising power, which is very important to treat dryness of the nasal mucosa, often associated with local allergic and inflammatory symptoms.

The compositions according to the invention can also contain water, preservatives, buffers, viscosity-modifying agents, suspending agents, pH-regulating agents and flavourings.

The compositions according to the invention are preferably administered in the form of a nasal spray.

In the compositions according to the invention, xyloglucans can be present in a wide concentration range which depends on the type of composition and the therapeutic indication for which they are intended.

The xyloglucan concentration range for a single dose is between 0.1 mg/dose and 10 mg/dose, preferably between 0.5 mg/dose and 2.5 mg/dose.

The formulations according to the invention are able to moisten dry mucous membranes and local microlesions caused by abundant rhinorrhoea.

The examples below are reference examples which are not in the scope of the claims.

The following nasal spray solutions were prepared:

### Example 1

| ***Ingredient*** | ***g*/*100 g*** |
|---|---|
| METHYLSULPHONYLMETHANE | 1.00000 |
| SALINE SOLUTION - 0.9% sodium chloride | q.s. for 100 |
| TAMARIND seed polysaccharides (GLYLOID 3S) | 1.00000 |
| SODIUM EDTA | 0.07500 |
| BORIC ACID (3% sol.) | 10.00000 |
| 14% NaOH SOLUTION | 0.19000 (pH=6.8) |
| TOTAL | 100.00 |

### Example 2

| ***Ingredient*** | ***g*/*100 g*** |
|---|---|
| METHYLSULPHONYLMETHANE | 1.00000 |
| SALINE SOLUTION - 0.9% sodium chloride | q.s. for 100 |
| XYLOGLUCAN | 0.5000 |
| GELATIN | 0.5000 |
| SODIUM EDTA | 0.07500 |
| BORIC ACID (3% sol.) | 10.00000 |
| TOTAL | 100.00 |

### Example 3

| ***Ingredient*** | ***g*/*100 g*** |
|---|---|
| METHYLSULPHONYLMETHANE | 1.00000 |
| SALINE SOLUTION - 0.9% sodium chloride | q.s. for 100 |
| XYLOGLUCAN | 0.5000 |
| GELATIN | 0.5000 |
| SODIUM EDTA | 0.07500 |
| BORIC ACID | 10.00000 |
| 14% NaOH SOLUTION | 0.19000 (pH=6.8) |
| TOTAL | 100.00 |

### Example 4

| ***COMPOSITION*** | ***g*/*15 ml*** |
|---|---|
| METHYLSULPHONYLMETHANE | 0.154 |
| XYLOGLUCAN | 0.154 |
| POTASSIUM SORBATE | 0.030 |
| SODIUM EDTA | 0.011 |
| BORIC ACID | 1.500 |
| SALINE SOLUTION | 13.48 |
| SODIUM HYDROXIDE SOLUTION 14% | 0.030 |
| TOTAL | 15.36 |

### Example 5

Two groups of 20 patients each, suffering from rhinosinusitis, were treated with the composition described in example 4 (TEST) or saline solution (PLACEBO). The treatment involved spraying the TEST and PLACEBO compositions twice a day. The treatment lasted for two weeks. The efficacy of the treatment was evaluated by means of a total score of the symptoms, comprising congestion, rhinorrhoea, itching and sneezing (TNSS, Total Nasal Symptom scores). The scores, evaluated at the beginning and end of the treatment with Student's t-test, were used to assign a rhinosinusitis score with the same statistical method. Nocturnal awakenings were also measured.

The results, set out in the table below, demonstrated that treatment with xyloglucans was more effective than saline solution.

**Group Statistics**

| | Treatment | N | Mean | Std Deviation | Std. Error Mean |
|---|---|---|---|---|---|
| TNSS at pre-study | TEST | 20 | 8,00 | ,000^{a} | ,000 |
| | PLACEBO | 20 | 8,00 | ,000^{a} | ,000 |
| Rhinosinusitis at pre-study | TEST | 20 | 6,40 | 1,501 | ,336 |
| | PLACEBO | 20 | 5,65 | 1,424 | ,319 |
| Nocturnal awakenings at | TEST | 20 | 2,20 | ,834 | ,186 |
| pre-study | PLACEBO | 20 | 2,20 | ,696 | ,156 |

**Group Statistics**

| | Treatment | N | Mean | Std. Deviation | Std. Error Mean |
|---|---|---|---|---|---|
| TNSS at post-study | TEST | 20 | 2,95 | 1,959 | ,438 |
| | PLACEBO | 20 | 4,40 | 2,563 | ,573 |
| Rhinosinusitis at post-study | TEST | 20 | 7,00 | 2,224 | ,497 |
| | PLACEBO | 20 | 6,05 | 1,504 | ,336 |
| Nocturnal awakenings at | TEST | 20 | 1,60 | 1,046 | ,234 |
| post-study | PLACEBO | 20 | 2,20 | ,834 | ,186 |

## Claims

1. Xyloglucans or extracts containing them in combination with pea protein and optionally with one or more active ingredients and/or excipients, wherein xyloglucans and pea protein are in 1:1 weight ratio, for use in the topical intranasal treatment of nasal congestion.

2. Xyloglucans or extracts containing them in combination with pea protein for use according to claim 1,
wherein the nasal congestion is associated with rhinitis and/or sinusitis.

3. Xyloglucans or extracts containing them in combination with pea protein for use according to claim 1,
wherein the active ingredient is an antibiotic, antiviral, anti-inflammatory and/or decongestant.

4. Xyloglucans or extracts containing them in combination with pea protein for use according to claims 1 to 3, able to moisten dry mucous membranes and local microlesions.

## Patentansprüche

1. Xyloglucane oder diese enthaltende Extrakte in Kombination mit Erbsenprotein und gegebenenfalls mit einem oder mehreren aktiven Wirkstoffen und/oder Exzipietenten, wobei die Xyloglucane und das Erbsenprotein in einem Gewichtsverhältnis von 1:1 vorliegen, zur Verwendung in der topischen intranasalen Behandlung von nasaler Kongestion.

2. Xyloglucane oder diese enthaltende Extrakte in Kombination mit Erbsenprotein für die Verwendung gemäß Anspruch 1, worin die nasale Kongestion mit einer Rhinitis und/oder Sinusitis assoziiert ist.

3. Xyloglucane oder diese enthaltende Extrakte in Kombination mit Erbsenprotein für die Verwendung gemäß Anspruch 1, worin der aktive Wirkstoff ein antibiotisches, antivirales, entzündungshemmendes und/oder abschwellendes Mittel ist.

4. Xyloglucane oder diese enthaltende Extrakte in Kombination mit Erbsenprotein für die Verwendung gemäß Ansprüche 1 bis 3, welche geeignet sind, trockene Schleimhäute oder lokale Mikroläsionen zu befeuchten.

## Revendications

1. Xyloglucanes ou extraits les contenant en combinaison avec une protéine du pois et éventuellement avec un ou plusieurs principes actifs et/ou excipients, dans lesquels les xyloglucanes et la protéine du pois sont dans un rapport en poids de 1 : 1, destinés à être utilisés dans le traitement intranasal topique de la congestion nasale.

2. Xyloglucanes ou extraits les contenant en combinaison avec une protéine de pois destinés à être utilisés selon la revendication 1, dans lesquels la congestion nasale est associée avec la rhinite et/ou la sinusite.

3. Xyloglucanes ou extraits les contenant en combinaison avec une protéine de pois destinés à être utilisés selon la revendication 1, dans lesquels le principe actif est un antibiotique, un antiviral, un anti-inflammatoire et/ou un décongestionnant.

4. Xyloglucanes ou extraits les contenant en combinaison avec une protéine de pois destinés à être utilisés selon les revendications 1 à 3, capables d'humidifier des membranes muqueuse sèches et des microlésions locales.
